# EUROPÄISCHE PATENTSCHRIFT

(11) **EP 2 800 515 B1**
(45) Veröffentlichungstag und Bekanntmachung des Hinweises auf die Patenterteilung: **04.05.2016**
(21) Anmeldenummer: 13706179.2
(22) Anmeldetag: 22.01.2013
(51) Int. Cl.: A61B 5/05, A61B 5/1455, A61B 5/15, A61B 5/153, A61B 5/154, A61B 5/157, A61B 5/145

(54) **BLUTENTNAHMERÖHRCHEN MIT INTEGRIERTER SENSOREINRICHTUNG**
BLOOD-SAMPLING TUBE WITH INTEGRATED SENSOR DEVICE
TUBE DE PRÉLÈVEMENT DE SANG MUNI D'UN DISPOSITIF DE DÉTECTION INTÉGRÉ

(30) Priorität: 14.02.2012 DE 102012202197
(43) Veröffentlichungstag der Anmeldung: 12.11.2014
(73) Patentinhaber: Siemens Aktiengesellschaft, 80333 München (DE)
(72) Erfinder: HAYDEN, Oliver, 91074 Herzogenaurach (DE)
(86) Internationale Anmeldenummer: PCT/EP2013/051107
(87) Internationale Veröffentlichungsnummer: WO 2013/120665

(56) Entgegenhaltungen:
- EP-A1- 2 236 077
- EP-A1- 2 281 507
- DE-A1- 2 543 576
- DE-A1- 2 927 048
- DE-B- 1 038 310
- US-A- 5 029 583
- US-A- 5 048 539
- US-A1- 2008 177 154
- US-A1- 2011 009 812

## Beschreibung

Die Erfindung betrifft ein Blutentnahmeröhrchen mit einer integrierten Sensoreinrichtung zur Messung einer biochemischen Funktion eines Fluids, insbesondere eines von einem Organismus entnommenen Fluids.

Blutentnahmeröhrchen sind weitverbreitet und dienen herkömmlicherweise als Probentransportbehälter, insbesondere zur Entnahme und Aufbereitung von Blutproben. Blutentnahmeröhrchen ermöglichen den Transport des entnommenen Blutes sowie eine kurzfristige Lagerung der entnommenen Proben. Ähnliche Systeme existieren auch für andere Körperfluide, insbesondere Urinproben.

Es gibt herkömmlicherweise zwei unterschiedliche Systeme, die Proben entweder durch Aspiration oder durch Unterdruck aufnehmen. Bei beiden Systemen existieren Röhrchen mit verschiedenen Zusätzen. Ein Blutentnahmeröhrchen mit einem Aspirationssystem hat in etwa den Aufbau einer Spritze. Durch Herausziehen eines Stempels entsteht ein Unterdruck, der die Entnahme des Fluids beschleunigt. Demgegenüber besteht bei einem Blutentnahmeröhrchen in einem Unterdrucksystem innerhalb des Blutentnahmeröhrchens bereits von vornherein ein Unterdruck. Wird das Blutentnahmeröhrchen auf einen mit einer Punktionskanüle verbundenen Adapter gesteckt, wird durch den vorhandenen Unterdruck das Fluid, insbesondere Blut, angesaugt. Das aufgenommene Blut kann bei beiden Systemen beispielsweise mit Citrat zur Lagerung oder zum Transport stabilisiert werden.

Bei herkömmlichen Systemen ist es notwendig, dass das entnommene Fluid, insbesondere das entnommene Blut, zur weiteren Analyse dem Blutentnahmeröhrchen entnommen wird, beispielsweise durch Pipettieren. Diese Vorgehensweise hat einige Nachteile. Zum einen bedarf es zur Entnahme des Fluids mithilfe eines Pipettiergerätes eines gewissen manuellen Geschicks der entnehmenden Person, sodass für diese Vorgehensweise nur entsprechend ausgebildetes Personal in Frage kommt. Weiterhin muss ein derartiges Pipettiergerät zur Entnahme des Fluids aus dem Blutentnahmeröhrchen vor Ort auch vorhanden sein. Dies bedeutet, dass die Person, die das Blut zur Analyse entnehmen möchte, über ein entsprechendes Pipettiergerät vor Ort verfügen muss, wobei dies allerdings beispielsweise in einer Wohnung eines Patienten nicht immer gewährleistet werden kann. Darüber hinaus ist das Entnehmen des Fluids aus dem Blutentnahmeröhrchen relativ mühsam und zeitaufwendig, wodurch sich die Analyse der biochemischen Funktionen des entnommenen Fluids verzögert. Mögliche Verbesserungen ergeben sich zwar durch die Verwendung von Blutentnahmevorrichtungen mit eingebauten Sensoreinrichtungen wie sie bspw. in DE2927048, EP2281507 sowie DE1038310 beschrieben werden. Derartige Vorrichtungen sind aber vergleichsweise aufwändig und kostenintensiv.

Es ist daher eine Aufgabe der vorliegenden Erfindung, ein Blutentnahmeröhrchen zu schaffen, welches eine schnelle und zuverlässige Erfassung einer biochemischen Funktion eines Fluids erlaubt und welches gleichzeitig einfach handhabbar ist.

Diese Aufgabe wird erfindungsgemäß durch ein Blutentnahmeröhrchen mit den in Patentanspruch 1 angegebenen Merkmalen gelöst.

Die Erfindung schafft ein Blutentnahmeröhrchen mit einer ersten Fluidkammer zur Aufnahme eines Fluids, wobei die erste Fluidaufnahmekammer mit mindestens einem Fluidkanal verbindbar ist, welcher eine Sensoreinrichtung zur Messung mindestens einer biochemischen Funktion des durch den Fluidkanal hindurchgeführten Fluids aufweist,
wobei die gemessene biochemische Funktion des Fluids von der Sensoreinrichtung über eine Datenschnittstelle des Blutentnahmeröhrchens auslesbar ist.

Bei einer möglichen Ausführungsform des erfindungsgemäßen Blutentnahmeröhrchens gelangt das durch den Fluidkanal hindurchgeführte Fluid in eine zweite Fluidaufnahmekammer, die mit einem Unterdruck beaufschlagbar ist.

Bei einer alternativen Ausführungsform ist der Fluidkanal direkt mit einem Unterdruck beaufschlagbar.

Bei einer möglichen Ausführungsform des erfindungsgemäßen Blutentnahmeröhrchens sind beide Fluidaufnahmekammern von dem Fluidkanal jeweils durch eine Berstscheibe oder durch ein Ventil getrennt.

Bei einer möglichen Ausführungsform des erfindungsgemäßen Blutentnahmeröhrchens ist auf das Blutentnahmeröhrchen eine Nadel aufsetzbar, über die eine Körperflüssigkeit, insbesondere venöses Blut oder Urin, aus einem Organismus entnehmbar ist.

Bei einer möglichen Ausführungsform des erfindungsgemäßen Blutentnahmeröhrchens ist in dem Fluidkanal eine Inkubationseinrichtung zur Zugabe von Reagenzien zu dem durch den Fluidkanal hindurchgeführten Fluid vorgesehen.

Bei einer weiteren möglichen Ausführungsform des erfindungsgemäßen Blutentnahmeröhrchens weist der zwischen der Inkubationseinrichtung und der Sensoreinrichtung liegende Fluidkanal ein vorgegebenes Fluidkanalvolumen auf, wobei dieses Fluidkanalvolumen sowie eine Strömungsgeschwindigkeit des durch den Fluidkanal hindurchströmenden Fluids eine einstellbare Reaktionszeit der von der Inkubationseinrichtung abgegebenen Reagenzien mit dem Fluid bestimmen.

Bei einer weiteren möglichen Ausführungsform des erfindungsgemäßen Blutentnahmeröhrchens hängt die Strömungsgeschwindigkeit des Fluids durch den Fluidkanal von einem einstellbaren Druckgefälle zwischen den beiden Fluidaufnahmekammern des Blutentnahmeröhrchens ab.

Bei einer möglichen Ausführungsform des erfindungsgemäßen Blutentnahmeröhrchens misst die Sensoreinrichtung die biochemische Funktion des Fluids kontaktfrei.

Bei einer möglichen Ausführungsform des erfindungsgemäßen Blutentnahmeröhrchens weist die Sensoreinrichtung mindestens ein magnetoelastisches Kapillarrohr auf, durch welches das zu untersuchende Fluid geführt wird.

Bei einer möglichen Ausführungsform des erfindungsgemäßen Blutentnahmeröhrchens ist eine Resonanzfrequenz des magnetoelastischen Kapillarrohres, welche von einer Oberflächenbeladung der Innenwandung des magnetoelastischen Kapillarrohres abhängt, zur Bestimmung der biochemischen Funktion des Fluids kontaktfrei auslesbar.

Bei einer weiteren Ausführungsform des erfindungsgemäßen Blutentnahmeröhrchens ermittelt die Sensoreinrichtung die biochemische Funktion des Fluids durch eine optische Transmissions- oder Streulichtmessung.

Bei einer weiteren möglichen Ausführungsform des erfindungsgemäßen Blutentnahmeröhrchens ist die Datenschnittstelle des Blutentnahmeröhrchens eine drahtlose Datenschnittstelle.

Bei einer weiteren möglichen Ausführungsform des erfindungsgemäßen Blutentnahmeröhrchens ist die Datenschnittstelle des Blutentnahmeröhrchens eine drahtgebundene Datenschnittstelle.

Bei einer weiteren möglichen Ausführungsform des erfindungsgemäßen Blutentnahmeröhrchens weist die Datenschnittstelle des Blutentnahmeröhrchens eine entnehmbare Datenspeicherkarte auf.

Bei einer möglichen Ausführungsform des erfindungsgemäßen Blutentnahmeröhrchens überträgt die Datenschnittstelle des Blutentnahmeröhrchens die gemessene biochemische Funktion des Fluids an ein externes Lesegerät.

Bei einer möglichen Ausführungsform des erfindungsgemäßen Blutentnahmeröhrchens handelt es sich bei dem mit der Datenschnittstelle des Blutentnahmeröhrchens verbundenen externen Lesegerät um ein mobiles Handgerät, insbesondere um ein Mobilfunkgerät.

Bei einer möglichen Ausführungsform des erfindungsgemäßen Blutentnahmeröhrchens handelt es sich bei dem Blutentnahmeröhrchen um ein Einwegprodukt.

Im Folgenden werden mögliche Ausführungsformen des erfindungsgemäßen Blutentnahmeröhrchens unter Bezugnahme auf die beigefügten Figuren näher beschrieben.

Es zeigen:
- Fig. 1: ein erstes Ausführungsbeispiel für ein erfindungsgemäßes Blutentnahmeröhrchen;
- Fig. 2: ein zweites Ausführungsbeispiel für ein erfindungsgemäßes Blutentnahmeröhrchen.

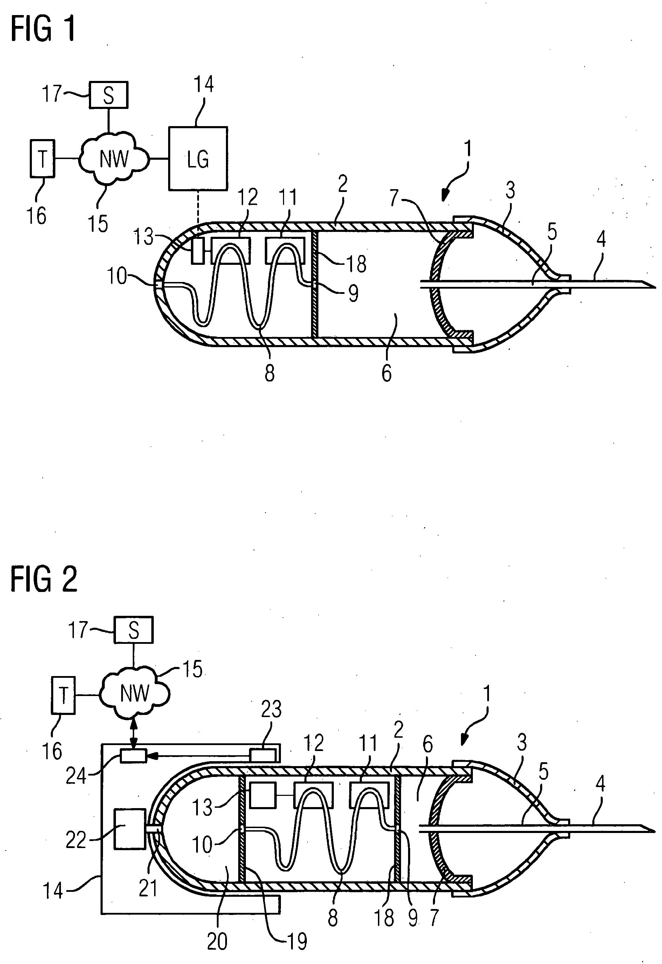

Wie man aus Fig. 1 erkennen kann, weist ein erfindungsgemäßes Blutentnahmeröhrchen 1 ein Gehäuse 2 auf, das beispielsweise aus einem transparenten Kunststoffmaterial bestehen kann. Auf das Gehäuse 2 ist eine Kappe 3 aufsetzbar, die eine Nadel 4 zur Entnahme eines Fluids F trägt. Die Nadel 4 eignet sich zur Entnahme eines Fluids F aus einem Organismus, wobei es sich bei dem Fluid F beispielsweise um venöses Blut oder um Urin handeln kann. Das entnommene Fluid F gelangt über eine Kanüle 5 in eine erste Fluidaufnahmekammer 6 des Blutentnahmeröhrchens 1. Die Kanüle 5 durchdringt dabei beispielsweise einen elastischen Gummi 7, der das Gehäuse 2 des Blutentnahmeröhrchens 1 dicht verschließt. Die erste Fluidaufnahmekammer 6 dient zur Aufnahme des entnommenen Fluids F, wobei die erste Fluidaufnahmekammer 6 mit mindestens einem Fluidkanal 8 verbindbar ist, beispielsweise durch eine Berstscheibe 9. Die Fluidaufnahmekammer bzw. das Kompartment 6 ist somit durch eine Berst- bzw. Dichtscheibe 9 von dem Fluidkanal 8 getrennt, wobei die Dichtscheibe bzw. die Berstscheibe durch Anlegen eines hohen negativen Druckes bzw. Unterdruckes geöffnet werden kann bzw. birst. Bei einer alternativen Ausführungsform kann anstelle der Berst- bzw. Dichtscheibe 9 auch ein steuerbares Ventil verwendet werden. Bei einer möglichen Ausführungsform ist dieses Ventil ein mikromechanisches Ventil, welches zum Öffnen und Schließen steuerbar ist. Bei der in Fig. 1 dargestellten Ausführungsform ist der Fluidkanal 8 mäanderförmig ausgebildet, wobei er an einem distalen Ende durch die Berstscheibe 9 von der ersten Fluidaufnahmekammer 6 getrennt ist. Am anderen Ende des mäanderförmigen Fluidkanals 8 kann eine weitere Berst- bzw. Dichtscheibe 10 vorgesehen sein, die das Gehäuse 2 des Blutentnahmeröhrchens nach außen abschließt, wobei diese Berst- bzw. Dichtscheibe 10 ebenfalls durch Anlegen eines negativen Druckes, d.h. Ansaugdruckes, geöffnet werden kann. Bei dem in Fig. 1 dargestellten Ausführungsbeispiel wird das in der Fluidaufnahmekammer 6 befindliche Fluid nach Bersten bzw. Öffnen der Dicht- bzw. Berstscheiben 9, 10 durch den Fluidkanal 8 geführt, wobei das Fluid F zunächst zu einer Inkubationseinrichtung 11 gelangt. Die Inkubationseinrichtung 11 ist dazu ausgelegt, Reagenzien zu dem durch den Fluidkanal 8 hindurchgeführten Fluid F hinzuzugeben, typischerweise biochemische Stoffe. Diese von der Inkubationseinrichtung 11 abgegebenen Reagenzien können mit dem hindurchströmenden Fluid F eine biochemische Reaktion eingehen. Nachdem die Reagenzien durch die Inkubationseinrichtung 11 dem hindurchströmenden Fluid F hinzugefügt wurden, gelangt das Fluid F nach einer gewissen Strecke zu einer Sensoreinrichtung 12, welche mindestens eine biochemische Funktion des durch den Fluidkanal 8 hindurchströmenden Fluids F misst. Die Sensoreinrichtung 12 kann sich beispielsweise auf einem Mikrofluidchip befinden. Bei dem hindurchströmenden Fluid F kann es sich beispielsweise um entnommenes venöses Blut handeln, wobei die Sensoreinrichtung 12 eine biochemische Funktion des Blutes misst beispielsweise dessen Gerinnungseigenschaften. Weiterhin kann eine Thrombozytenfunktion des entnommenen venösen Blutes durch die Sensoreinrichtung 12 gemessen werden. Die von der Sensoreinrichtung 12 gemessene biochemische Funktion des Fluids F ist über eine Datenschnittstelle 13 des Blutentnahmeröhrchens 1 auslesbar. Die Datenschnittstelle 13 ist, wie in Fig. 1 dargestellt, direkt mit der Sensoreinrichtung 12 verbunden. Bei der in Fig. 1 dargestellten Ausführungsform ist die Datenschnittstelle 12 eine drahtlose Datenschnittstelle, bei der Daten drahtlos von der Datenschnittstelle 13 durch ein externes Lesegerät 14 auslesbar sind. Alternativ kann es sich bei der Datenschnittstelle 13 auch um eine drahtgebundene Datenschnittstelle handeln, wobei Daten über ein Kabel durch das in Fig. 1 dargestellte Lesegerät 14, auslesbar sind. Bei einer weiteren möglichen Ausführungsform wird die Datenschnittstelle 13 durch eine entnehmbare Datenspeicherkarte gebildet, die dem Blutentnahmeröhrchen 1 entnehmbar ist. Das Lesegerät 14 ist bei dem in Fig. 1 dargestellten Ausführungsbeispiel über ein Datennetzwerk 15, beispielsweise das Internet, mit einem Terminal 16 und einem Server 17 verbunden, der beispielsweise Zugriff auf eine medizinische Datenbank hat. Auf diese Weise können die durch die Sensoreinheit 12 ermittelten Daten hinsichtlich der biochemischen Funktion des Fluids F auf das Lesegerät 14 und das Datennetzwerk 15 zu einem entfernten Terminal 16 unmittelbar übertragen werden. Beispielsweise kann das von einem Sanitäter beispielsweise in einer Wohnung eines Patienten von dem Patienten entnommene Blut durch die Sensoreinrichtung 12 hinsichtlich einer biochemischen Funktion analysiert werden und das Analyseergebnis über das Netzwerk an einen entfernten Terminal 16 übertragen werden, an dem eine medizinisch qualifizierte Person, beispielsweise ein Laborarzt, sitzt. Weiterhin können die übertragenen Daten direkt in einer Datenbank des in Fig. 1 dargestellten Servers 17 zur weiteren Untersuchung abgelegt werden.

Wie in Fig. 1 dargestellt, weist der Fluidkanal 8 zwischen der Inkubationseinrichtung 11 und der Sensoreinrichtung 12 eine vorgegebene Strecke auf und bildet somit ein vorgegebenes Fluidkanalvolumen. Dieses Fluidkanalvolumen sowie eine Strömungsgeschwindigkeit des durch den Fluidkanal 8 hindurchströmenden Fluids F bestimmen eine einstellbare Reaktionszeit der von der Inkubationseinrichtung 11 abgegebenen Reagenzien mit dem aus dem Organismus entnommenen Fluid. Die Strömungsgeschwindigkeit des Fluids wird dabei von einem einstellbaren Druckgefälle (AP) in dem Fluidkanal 8 bestimmt. Auf diese Weise kann eine ausreichend lange Reaktionszeit T mit den zugegebenen Reagenzien erreicht werden.

Bei einer bevorzugten Ausführungsform wird die biochemische Funktion des Fluids F mithilfe der Sensoreinrichtung 12 kontaktfrei gemessen. Bei einer möglichen Ausführungsform weist die Sensoreinrichtung 12 mindestens ein magnetoelastisches Kapillarrohr auf, durch die das zu untersuchende Fluid F geführt wird. Bei einer möglichen Ausführungsform ist dabei eine Resonanzfrequenz fR des magnetoelastischen Kapillarrohres messbar und auslesbar. Diese Resonanzfrequenz fR hängt von einer Oberflächenbeladung der Innenwandung des magnetoelastischen Kapillarrohres der Sensoreinrichtung 12 ab, wobei mithilfe der erfassten Resonanzfrequenz fR eine biochemische Funktion des Fluids, beispielsweise eine Thrombozytenfunktion von Blut, köntaktfrei ermittelbar ist. Bei einer alternativen Ausführungsform wird die biochemische Funktion des Fluids durch die Sensoreinrichtung 12 durch eine optische Transmissions- oder Streulichtmessung ermittelt.

Das in Fig. 1 dargestellte Blutentnahmeröhrchen 1 kann als Einwegprodukt ausgebildet sein, d.h. nach Ermittlung der biochemischen Funktion wird das Blutentnahmeröhrchen 1 nicht wiederverwendet. Der negative Druck, welcher beispielsweise die in Fig. 1 dargestellten Berstscheiben 9, 10 zum Bersten bringt, kann auf verschiedene Weise erzeugt werden. Beispielsweise kann an der Berstscheibe 10 von außen ein Unterdruck angelegt werden, welcher zunächst die Berstscheibe 10 und anschließend die Berstscheibe 9 zum Bersten bringt, sodass das in der Fluidaufnahmekammer 6 befindliche Fluid F durch das Fluidrohr 8 zu der geborstenen Scheibe 10 hin fließt.

Bei der in Fig. 1 dargestellten Ausführungsform weist das Blutentnahmeröhrchen einen Fluidkanal 8 auf. Bei einer alternativen Ausführungsform können auch mehrere Fluidkanäle 8 parallel vorgesehen sein, wobei jeder Fluidkanal 8 über eine Inkubationseinrichtung 11 und eine Sensoreinrichtung 12 verfügt. Das durch die geborstene Scheibe 10 austretende Fluid F kann durch eine weitere Fluidaufnahmekammer bzw. ein Waste Compartment aufgenommen werden.

Fig. 2 zeigt ein Ausführungsbeispiel, bei dem das Blutentnahmeröhrchen 1 über eine integrierte zweite Fluidaufnahmekammer bzw. ein Waste Compartment 20 verfügt. Wie man aus den Fig. 1, 2 erkennen kann, sind die in dem dargestellten Ausführungsbeispiel vorgesehenen Berstscheiben 9, 10 in Trennwandungen 18, 19 angeordnet. Nach Bersten der Berstscheibe 10 fließt das in der ersten Fluidkammer 6 befindliche Fluid F durch den Fluidkanal 8 in die zweite Fluidaufnahmekammer 20, die sich ebenfalls in dem Gehäuse 2 des Blutentnahmeröhrchens 1 befindet. Über eine Öffnung 21, die sich im Gehäuse 2 des Blutentnahmeröhrchens 1 befindet, kann die zweite Fluidaufnahmekammer 20 bzw. das Waste Compartment an eine Unterdruckeinrichtung 22 eines externen Gerätes angeschlossen werden, um einen Unterdruck in dem Waste Compartment 20 aufzubauen, welcher seinerseits die beiden Berstscheiben 9, 10 zum Bersten bringt, sodass das Fluid F aus der ersten Fluidaufnahmekammer 6 über den Fluidkanal 8 in die zweite Fluidaufnahmekammer 20 fließt. Das hindurchfließende Fluid F wird dabei durch die Sensoreinrichtung 12 zur Ermittlung einer biochemischen Funktion des Fluids analysiert. Durch das Vorsehen der zweiten Fluidaufnahmekammer 20 wird erreicht, dass das Blutentnahmeröhrchen 1 ein geschlossenes System bildet, sodass die untersuchende Person mit dem entnommenen Fluid F, beispielsweise Blut, nicht in Kontakt tritt. Dies ist von Vorteil, da das entnommene Fluid F, beispielsweise Blut, potenziell infektiös sein kann. Bei der in Fig. 2 dargestellten Ausführungsform ist die Unterdruckeinrichtung 22 in dem Lesegerät 14 mitintegriert ,wobei das Lesegerät 14 über eine integrierte Datenschnittstelle 23 verfügt, die drahtgebunden oder drahtlos Daten aus der Datenschnittstelle 13 des Blutentnahmeröhrchens 1 ausliest. Die ausgelesenen Daten werden bei der in Fig. 2 dargestellten Ausführungsform direkt über eine weitere Datenschnittstelle 24 des Lesegerätes 14 über das Netzwerk 15 an dem entfernten Terminal 16 und/oder den entfernten Server 17 übertragen. Bei dem in Fig. 2 dargestellten Ausführungsbeispiel ist das Blutentnahmeröhrchen 1 in das Lesegerät 14 einsetzbar, wobei das Lesegerät 14 zu-. sätzlich dafür sorgt, dass ein entsprechender Unterdruck an das eingesetzte Blutentnahmeröhrchen 1 angelegt wird. Nach der Entnahme und Analyse des Fluids F kann das Blutentnahmeröhrchen 1 zusätzlich gelagert werden. Dies ist insbesondere dann nützlich, wenn sich bei der Analyse der biochemischen Funktion des Fluids F eine Auffälligkeit ergibt, die durch eine Zweitmessung verifiziert werden soll. Bei einer möglichen Ausführungsform kann in dem Blutentnahmeröhrchen 1 eine Stabilisierungssubstanz, beispielsweise Citrat, zur Lagerung des entnommenen Fluids F vorgefüllt sein.

## Patentansprüche

1. Blutentnahmeröhrchen (1) mit
einer ersten Fluidaufnahmekammer (6) zur Aufnahme eines Fluids, **dadurch gekennzeichnet, dass**
die erste Fluidaufnahmekammer (6) mit mindestens einem Fluidkanal (8) des Blutentnahmeröhrchens (1) verbindbar ist, welcher eine Sensoreinrichtung (12) zur Messung mindestens einer biochemischen Funktion des durch den Fluidkanal (8) hindurchgeführten Fluids aufweist,
wobei die gemessene biochemische Funktion des Fluids von der Sensoreinrichtung (12) über eine Datenschnittstelle (13) des Blutentnahmeröhrchens (1) auslesbar ist.

2. Blutentnahmeröhrchen nach Anspruch 1,
wobei das durch den Fluidkanal (8) hindurchgeführte Fluid in eine zweite Fluidaufnahmekammer (20) gelangt, die mit einem Unterdruck beaufschlagbar ist.

3. Blutentnahmeröhrchen nach Anspruch 2,
wobei die beiden Fluidaufnahmekammern (6,20) von dem Fluidkanal (8) jeweils durch eine Berstscheibe (9,10) oder durch ein Ventil getrennt sind.

4. Blutentnahmeröhrchen nach einem der vorangehenden Ansprüche 1 bis 3,
wobei auf das Blutentnahmeröhrchen (1) eine Nadel (4) aufsetzbar ist, über die eine Körperflüssigkeit, insbesondere venöses Blut oder Urin, aus einem Organismus entnehmbar ist.

5. Blutentnahmeröhrchen nach einem der vorangehenden Ansprüche 1 bis 4,
wobei in dem Fluidkanal (8) eine Inkubationseinrichtung (11) zur Zugabe von Reagenzien zu dem durch den Fluidkanal (8) hindurchgeführten Fluid vorgesehen ist.

6. Blutentnahmeröhrchen nach Anspruch 5,
wobei zwischen der Inkubationseinrichtung (11) und der Sensoreinrichtung (12) der Fluidkanal (8) ein vorgegebenes Fluidkanalvolumen aufweist, wobei dieses Fluidkanalvolumen sowie eine Strömungsgeschwindigkeit des durch den Fluidkanal (8) hindurchströmenden Fluids eine einstellbare Reaktionszeit der von der Inkubationseinrichtung (11) abgegebenen Reagenzien mit dem Fluid bestimmen.

7. Blutentnahmeröhrchen nach Anspruch 6,
wöbei die Strömungsgeschwindigkeit des Fluids durch den Fluidkanal (8) von einem einstellbaren Druckgefälle zwischen den beiden Fluidaufnahmekammern (6,20) des Blutentnahmeröhrchens (1) abhängt.

8. Blutentnahmeröhrchen nach einem der vorangehenden Ansprüche 1 bis 7,
wobei die Sensoreinrichtung (12) die biochemische Funktion des Fluids kontaktfrei misst.

9. Blutentnahmeröhrchen nach Anspruch 8,
wobei die Sensoreinrichtung (12) mindestens ein magnetoelastisches Kapillarrohr aufweist, durch die das zu untersuchende Fluid geführt wird.

10. Blutentnahmeröhrchen nach Anspruch 9,
wobei eine Resonanzfrequenz des magnetoelastischen Kapillarrohres, welche von einer Oberflächenbeladung der Innenwandung des magnetoelastischen Kapillarrohres abhängt, zur Bestimmung der biochemischen Funktion des Fluids kontaktfrei auslesbar ist.

11. Blutentnahmeröhrchen nach Anspruch 8,
wobei die Sensoreinrichtung (12) die biochemische Funktion des Fluids durch eine optische Transmissions- oder Streulichtmessung ermittelt.

12. Blutentnahmeröhrchen nach einem der vorangehenden Ansprüche 1 bis 11,
wobei die Datenschnittstelle (13) des Blutentnahmeröhrchens eine drahtlose oder drahtgebundene Datenschnittstelle oder eine aus dem Blutentnahmeröhrchen (1) entnehmbare Datenspeicherkarte aufweist.

13. Blutentnahmeröhrchen nach einem der vorangehenden Ansprüche 1 bis 12,
wobei die Datenschnittstelle (13) des Blutentnahmeröhrchens (1) die gemessene biochemische Funktion des Fluids an ein externes Lesegerät (14) überträgt.

14. Blutentnahmeröhrchen nach Anspruch 13,
wobei das mit der Datenschnittstelle verbundene externe Lesegerät (14) ein mobiles Handgerät, insbesondere ein Mobilfunkgerät, ist.

15. Blutentnahmeröhrchen nach einem der vorangehenden Ansprüche 1 bis 14,
wobei das Blutentnahmeröhrchen (1) ein Einwegprodukt ist.

## Claims

1. Blood sampling tube (1) comprising
a first fluid receiving chamber (6) for receiving a fluid, **characterized in that** the first fluid receiving chamber (6) can be connected to at least one fluid channel (8) of the blood sampling tube (1) comprising a sensor device (12) for measuring at least one biochemical function of the fluid passed through the fluid channel (8),
wherein the measured biochemical function of the fluid can be read out from the sensor device (12) via a data interface (13) of the blood sampling tube (1).

2. Blood sampling tube according to Claim 1,
wherein the fluid passed through the fluid channel (8) reaches a second fluid receiving chamber (20) to which negative pressure can be applied.

3. Blood sampling tube according to Claim 2,
wherein the two fluid receiving chambers (6, 20) are separated in each case from the fluid channel (8) by a rupture disk (9, 10) or by a valve.

4. Blood sampling tube according to one of the preceding Claims 1 to 3,
wherein a needle (4) can be placed onto the blood sampling tube (1), by means of which needle a bodily fluid, in particular venous blood or urine, can be withdrawn from an organism.

5. Blood sampling tube according to one of the preceding Claims 1 to 4,
wherein an incubation device (11) for adding reagents to the fluid passed through the fluid channel (8) is provided in the fluid channel (8).

6. Blood sampling tube according to Claim 5,
wherein the fluid channel (8) has a predetermined fluid channel volume between the incubation device (11) and the sensor device (12), wherein this fluid channel volume and a flow velocity of the fluid flowing through the fluid channel (8) determine an adjustable reaction time of the reagents, dispensed by the incubation device (11), with the fluid.

7. Blood sampling tube according to Claim 6,
wherein the flow velocity of the fluid through the fluid channel (8) depends upon an adjustable pressure drop between the two fluid receiving chambers (6, 20) of the blood sampling tube (1).

8. Blood sampling tube according to one of the preceding Claims 1 to 7,
wherein the sensor device (12) measures the biochemical function of the fluid contactlessly.

9. Blood sampling tube according to Claim 8,
wherein the sensor device (12) comprises at least one magnetoelastic capillary tube, through which the fluid to be examined is passed.

10. Blood sampling tube according to Claim 9,
wherein a resonant frequency of the magnetoelastic capillary tube, which depends on a surface load on the inner wall of the magnetoelastic capillary tube, can be read out contactlessly for determining the biochemical function of the fluid.

11. Blood sampling tube according to Claim 8,
wherein the sensor device (12) establishes the biochemical function of the fluid by an optical transmission or stray light measurement.

12. Blood sampling tube according to one of the preceding Claims 1 to 11,
wherein the data interface (13) of the blood sampling tube comprises a wireless or wired data interface or a data memory card which can be removed from the blood sampling tube (1).

13. Blood sampling tube according to one of the preceding Claims 1 to 12,
wherein the data interface (13) of the blood sampling tube (1) transmits the measured biochemical function of the fluid to an external readout instrument (14).

14. Blood sampling tube according to Claim 13,
wherein the external readout instrument (14) connected to the data interface is a mobile hand-held device, in particular a mobile communications device.

15. Blood sampling tube according to one of the preceding Claims 1 to 14,
wherein the blood sampling tube (1) is a disposable product.

## Revendications

1. Tube de prélèvement de sang (1) comprenant
une première chambre de réception de fluide (6) destinée à recevoir un fluide, **caractérisé en ce que**
la première chambre de réception de fluide (6) peut communiquer avec au moins un canal de fluide (8) du tube de prélèvement de sang (1), qui présente un dispositif de détection (12) pour mesurer au moins une fonction biochimique du fluide traversant le canal de fluide (8),
la fonction biochimique mesurée du fluide pouvant être lue par le dispositif de détection (12) via une interface de données (13) du tube de prélèvement de sang (1).

2. Tube de prélèvement de sang selon la revendication 1,
le fluide traversant le canal de fluide (8) parvenant dans une seconde chambre de réception de fluide (20) pouvant être mise en dépression.

3. Tube de prélèvement de sang selon la revendication 2,
les deux chambres de réception de fluide (6, 20) étant séparées du canal de fluide (8) par un disque de rupture (9, 10) ou par une soupape.

4. Tube de prélèvement de sang selon l'une des revendications précédentes 1 à 3,
une aiguille (4) pouvant être placée sur le tube de prélèvement de sang (1), par laquelle un fluide organique, en particulier du sang veineux ou de l'urine, peut être prélevé sur un organisme.

5. Tube de prélèvement de sang selon l'une des revendications précédentes 1 à 4,
un dispositif d'incubation (11) étant prévu dans le canal de fluide (8), servant à ajouter des réactifs au fluide traversant le canal de fluide (8).

6. Tube de prélèvement de sang selon la revendication 5,
le canal de fluide (8) présentant entre le dispositif d'incubation (11) et le dispositif de détection (12) un volume de canal de fluide prédéfini, ledit volume ainsi qu'une vitesse d'écoulement du fluide traversant le canal de fluide (8) déterminant un temps de réaction réglable des réactifs distribués par le dispositif d'incubation (11) avec le fluide.

7. Tube de prélèvement de sang selon la revendication 6,
la vitesse d'écoulement du fluide à travers le canal de fluide (8) dépendant d'un gradient de pression réglable entre les deux chambres de réception de fluide (6, 20) du tube de prélèvement de sang (1).

8. Tube de prélèvement de sang selon l'une des revendications précédentes 1 à 7,
le dispositif de détection (12) mesurant la fonction biochimique du fluide sans contact.

9. Tube de prélèvement de sang selon la revendication 8,
le dispositif de détection (12) comprenant au moins un tube capillaire magnéto-élastique traversé par le fluide à analyser.

10. Tube de prélèvement de sang selon la revendication 9,
une fréquence de résonance du tube capillaire magnéto-élastique dépendant d'une charge superficielle de la paroi interne du tube capillaire magnéto-élastique pouvant être lue sans contact pour déterminer la fonction biochimique du fluide.

11. Tube de prélèvement de sang selon la revendication 8,
le dispositif de détection (12) déterminant la fonction biochimique du fluide par une mesure optique de transmission ou de lumière diffusée.

12. Tube de prélèvement de sang selon l'une des revendications précédentes 1 à 11,
l'interface de données (13) du tube de prélèvement de sang comprenant une interface de données sans fil ou filaire ou une carte de stockage de données pouvant être retirée du tube de prélèvement de sang (1).

13. Tube de prélèvement de sang selon l'une des revendications précédentes 1 à 12,
l'interface de données (13) du tube de prélèvement de sang (1) transmettant la fonction biochimique mesurée du fluide à un lecteur externe (14).

14. Tube de prélèvement de sang selon la revendication 13,
le lecteur externe (14) relié à l'interface de données étant un appareil portatif mobile, en particulier un appareil de téléphonie mobile.

15. Tube de prélèvement de sang selon l'une des revendications précédentes 1 à 14,
le tube de prélèvement de sang (1) étant un produit jetable.
